# EUROPEAN PATENT APPLICATION

(11) **EP 3 443 904 A1**
(43) Date of publication of application: **20.02.2019**
(21) Application number: 17186189.1
(22) Date of filing: 14.08.2017
(51) Int. Cl.: A61B 6/00

(54) **MULTI-SPOT X-RAY IMAGING**

(71) Applicant: Koninklijke Philips N.V., 5656 AE Eindhoven (NL)
(72) Inventor: BEHLING, Rolf Karl Otto, 5656 AE Eindhoven (NL); KOEHLER, Thomas, 5656 AE Eindhoven (NL)
(74) Representative: de Haan, Poul Erik

(57) **Abstract**

The present invention relates to a multi-spot X-ray device, a multi-spot X-ray imaging system and a method for imaging an object by use of a multi-spot X-ray device. The multi-spot X-ray device comprises a multi-spot array, a multi-part absorbing grating, and a first and second grating. The object is arranged between the multi-part absorbing grating and the first grating or the first grating and the second grating. The multi-part absorbing grating provides at least two parts with a phase offset relative to each other.

## Description

### FIELD OF THE INVENTION

The present invention relates to a multi-spot X-ray device, a multi-spot X-ray imaging system and a method for imaging an object by use of a multi-spot X-ray device.

### BACKGROUND OF THE INVENTION

The use of multiple focal spots in an X-ray source for enhancing coverage of the region of interest with a limited size of the image receptor has been proposed before. The size and costs of a detector as well as of other components of the imaging chain, e.g. anti-scatter grids, could be thus reduced. WO 2008/056299 A1 describes a multiple focal spot X-ray tube, which is adapted to generate an X-ray beam from at least two different focal spots positions.

In conventional X-ray computed tomography (CT) the three-dimensional distribution of the X-ray absorption coefficient is detected. An internal structure of an object, such as a human organ, can be determined. When using a Talbot-Lau interferometer, differential phase contrast (DCPI) tomography can be realized. Simultaneously, dark field imaging (DFI) providing absorption, phase shift and scattering images can be retrieved. For this, a conventional X-ray source, a Talbot-Lau interferometer and a conventional digital X-ray detector is utilized.

### SUMMARY OF THE INVENTION

There may thus be a need to enhance the imaging system, given the fixed size of the X-ray detector and the gratings of the Talbot-Lau interferometer for DPCI and DFI. The object of the present invention is solved by the subject-matter of the independent claims; further embodiments are incorporated in the dependent claims. It should be noted that the following described aspects of the invention apply also for a multi-spot X-ray device, a multi-spot X-ray imaging system, an according method and vice versa.

Computed tomography (CT) is a standard technique for radiology diagnosis. There has been an evolution to ever wider detector arrays that are able to cover whole organs with a single circular gantry sweep. A CT system may be used, providing two X-ray sources, which may be mounted on a gantry, aligned at about the same angular position and alternatively emitting X-rays which traverse an object to be imaged. A detector detects the radiation emitted by the two x-ray sources and generates composite data indicating the detected radiation. The composite data are reconstructed to generate one or more images.

When using two or more X-ray sources for imaging of an object, a single projection of the object would be split into multiple projections, which are then electronically overlaid to produce the final image of the object.

According to the present invention, a multi-spot X-ray device is provided. The multi-spot device comprises: a multi-spot array providing at least two focal spots configured to emit X-rays, a multi-part absorbing grating configured to transmit the X-rays through at least one of its parts, a first grating and a second grating, and an object-receiving region arranged between the multi-part absorbing grating and the first grating or the first grating and the second grating. At least two parts of the parts of the multi-part absorbing grating are provided with a phase offset relative to each other.

In some examples, the multi-spot X-ray device comprises a multi-spot array providing multiple focal spots. The multi-spot array comprises at least two focal spots. The multiple focal spots can be arranged linearly or in a cross-shaped arrangement. In an example, the focal spots have the same distance from the multi-part absorbing grating. The focal spots are configured to emit X-rays. The focal spots emit X-rays in a subsequent manner. This means, that only one focal spot emits X-rays at a time, so that only these X-rays are transmitted by the object and the gratings. In some examples, the focal spots are configured to emit coherent X-rays. In some examples, different X-ray spectra are emitted from different X-ray sources.

The multi-part absorbing grating provides at least two parts. Each part of the multi-part absorbing grating is configured to transmit the X-rays. The X-rays emitted from a focal spot are transmitted through a part of the parts of the multi-part absorbing grating.

The object to be imaged is arranged in the object-receiving region. The object-receiving region can be arranged between the multi-part absorbing grating and the first grating. In some examples, the object-receiving region is arranged between the first grating and the second grating. The first and second grating are provided as either a non-absorbing or an absorbing grating. The multi-part absorbing grating provides a phase offset relative to the first grating and/or second grating. In some examples, the arrangement of the grating provides an arrangement as for Talbot-Lau interferometry. Such arrangement provides differential phase contrast imaging (DPCI) and dark-field imaging (DFI).

At least two parts of the parts of the multi-part absorbing grating are provided with a phase offset relative to each other. Thus, stepping movements of the multi-part gratings are reduced at least by half compared to conventional stepping methods where an absorbing grating is provided, having a uniform grating. Also, the stepping speed of the multi-part grating is thus reduced by half. This especially relevant for CT imaging, for example, where the gantry movements are at least reduced by half providing an improved imaging technique with an enlarged field of view at the same time.

According to an example, the at least two focal spots of the multi-spot array are arranged facing at least a first and a second part, respectively, of the multi-part absorbing grating. The focal spots are arranged so that each focal spot faces one of the two parts of the multi-part absorbing grating. In an example, where the parts are arranged one above the other, when seen in the direction of the transmitted X-rays, also the two focal spots are arranged on top of each other. Thus, a rather simplified but highly effective multi-spot X-ray device can be provided. In a device providing two or more focal spots the field of view is enlarged in one direction. This direction is defined by the arrangement of the focal spots. For example, two focal spots arranged next to each other, when seen in beam direction, enlarge the field of view in a plane defined by the two focal spots and a center of the object-receiving region. Two focal spots arranged on top of each other, when seen in beam direction, enlarge the field of view in a corresponding plane. Thus, the arrangement of the focal spots can define the field of view. In an example with four focal spots the field of view is enlarged in two directions. An arrangement of three focal spots, providing a center focal spot, can avoid parallax effects in case a small field of view suffices.

According to an example, the focal spots are configured to alternatingly or sequentially emit X-rays. Only one part of the multi-part grating is illuminated at a time. In an example with two or three focal spots the X-rays are emitted by one of the focal spots and one part of the multi-part grating is illuminated and is transmitting the X-rays. The other focal spot(s) is (are) off and corresponding part(s) of the multi-part grating remains non-illuminated.

According to an example, the phase offset between the parts of the multi-part absorbing grating is between 0 and 180 degrees. In some examples 90, 120 or 180 is a preferred phase offset. Such phase offset between two or more parts of the multi-part absorbing grating or the separate absorbing gratings provides a significant reduction of the stepping movement compared to the stepping of an absorbing grating as known from conventional devices. In some examples, the stepping movements are reduced by half, according to the phase offset.

According to an example, the multi-part absorbing grating is configured to be rotated. The rotation is performed around an axis, so that the centre of rotation is located outside the grating area. By rotating the multi-part grating its projected shape can be varied from being rectangular, trapezoidal up to triangular.

According to an example, at least one of the parts of the multi-part absorbing grating is provided as separate absorbing grating. Each separate grating can be moved, rotated or stepped during the integration period of another part of the multi-part grating. In other words, the non-illuminated separate absorbing grating (non-illuminated separate part of the multi-part absorbing grating) can be moved anywhere during the integration period of an illuminated separate absorbing grating (illuminated part of the multi-part absorbing grating). The illuminated grating is the transmitting grating. This provides far less demands for the synchronization of the stepping and the individual integration period(s) of the illuminated grating or illuminated part of the multi-part absorbing grating. Furthermore, also the demands for the stepping speed or rotation speed for the gratings can be heavily reduced, e.g. the stepping speed can be easily reduced by half.

According to an example, the multi-spot array or the at least two focal spots are configured to be arranged, so that the X-rays emitted by one of the focal spots illuminates one of the parts of the multi-part absorbing grating. The multi-spot array can be arranged so that all or at least one part of the focal spots precisely face a part of the multi-part absorbing grating. In some examples, at least a part of the focal spots are movably arranged, so that a precise alignment of the focal spot and the part, which is illuminated, is provided.

According to the present invention, also a multi-spot X-ray imaging system is provided. The system comprises a source configured to provide at least one X-ray beam, a multi-spot X-ray device comprising multiple focal spots and a multi-part absorbing grating and a detector configured to receive X-rays emitted from at least a part of the multiple focal spots and transmitted at least through one of parts of the multi-part absorbing grating. The system further comprises a processing unit configured to integrate data signals provided by the detector corresponding to X-rays emitted by the multiple focal spots and to produce an image of an object by overlaying the integrated data signals. At least two of the parts of the multi-part absorbing grating are provided with a phase offset relative to each other. In some examples, the detector is a digital detector. The detector may comprise one or several detectors. The source may be configured to provide two, three or more X-ray beams. In some examples, different X-ray spectra are emitted from different X-ray sources. This is provided by different X-ray tube voltages and/or filtration techniques.

According to an aspect, a method for imaging an object by use of a multi-spot X-ray device is provided. The method comprises the following steps: emitting X-rays subsequently from at least two focal spots of a multi-spot array; passing the X-rays through at least one of the parts of a multi-part absorbing grating; transmitting the X-rays through the object, a first grating and a second grating; and integrating data signals provided by a detector corresponding to X-rays subsequently emitted by the at least two focal spots. At least two of the parts of the multi-part grating are provided with a phase offset relative to each other. Only one focal spot emits X-rays at a time. The other focal spots of the multi-spot array is(are) off. The emitted X-rays pass through one of the grating parts provided by the multi-part absorbing grating. After the data signals provided by the detector are integrated for the X-rays received by the detector from one of the focal spots, one of the remaining focal spots emits X-rays which pass through one of the remaining parts of the multi-part absorbing grating. The illuminated part of the multi-part absorbing grating provides a phase offset compared to the before illuminated part of the multi-part absorbing grating. Also, in some examples, the X-rays emitted by two different focal spots can illuminate the same grating part, as long as the subsequent illuminated part provides a phase offset relative to the previous illuminated part of the multi-part absorbing grating.

According to an example, the method comprises stepping the multi-part absorbing grating after integration of the data signals provided by the detector corresponding to the X-rays emitted by the at least two focal spots. Such method provides reduced stepping speed and reduced stepping steps. After the data signals of all X-rays which have been emitted by the at least two focal spots are integrated, the multi-part absorbing grating is stepped or moved, e.g. transverse to the direction of the emitted X-rays, so that another phase of the multi-part absorbing grating relative to the first and second grating is provided. Each integration of the data signals is performed during a corresponding integration period. In some examples with more than two focal spots, e.g. three focal spots, all three or more focal spots emit X-rays which pass through the corresponding part of the multi-part absorbing grating, are transmitted through the object and the first and second grating. The X-rays received by the detector are integrated during an integration period for each focal spot. The multi-part grating is then stepped after completion of integration. In other words, there is no need for stepping the absorbing grating after each integration period. The multi-part absorbing grating remains in position as long as the focal spots are emitting X-rays in an alternating or sequential manner. After all focal spots, which can also be only a part of the provided focal spots, emitted X-rays illuminating a corresponding part of the parts of the multi-part absorbing grating, the multi-part absorbing grating is moved, i.e. stepped. According to an example, the method comprises stepping the multi-part absorbing grating by rotation. Such method provides reduced motion or rotation speed of the multi-part absorbing grating.

According to an example, at least one of the parts of the multi-part absorbing grating is provided as separate absorbing grating and wherein the at least one part is being stepped during integration of the data signals provided by the X-rays passing another part of the multi-part absorbing grating. This provides alternating illumination of the separate absorbing grating. Each grating is moved during the integration time of the other grating. Demands for synchronization and stepping step or rotation is thus significantly reduced.

According to an example, during transmission of X-rays from one of the focal spots only one of the parts of the multi-part absorbing grating is illuminated. This provides, that the phase offset between the parts of the multi-part grating can be maintained.

A primary use of the device, system and method as provided by the invention is CT imaging, where the use of the multi-part absorbing grating along with a multi-spot array and first and second gratings, provides an imaging technique, which takes into account a fixed size of the X-ray detector and of the gratings for DPCI and DFI and simultaneously reduced the motion of the multi-part absorbing grating by reduction of the stepping rate. A further use of the device, system and method as provided by the invention is for 2D imaging, where parallax effects could be accepted, e.g. if small spatial resolution suffices.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 a multi-spot X-ray imaging system comprising a multi-spot X-ray device;
Fig. 2 a method for imaging an object by use of a multi-spot X-ray device;
Fig. 3 an example of stepping a multi-part absorbing grating providing a single part.
Fig. 4 a multi-part absorbing grating providing a phase offset of 180 degrees and a phase offset of 120 degrees;
Fig. 5 a method for imaging an object including stepping of a multi-part absorbing grating;
Fig. 6 stepping and integrating steps for multi-part absorbing grating providing a phase offset of 90 degrees;
Fig. 7 a multi-part absorbing grating provided as two separate gratings;
Fig. 8 a method for imaging an object by using the multi-part absorbing grating of Fig. 7;
Fig. 9 stepping and integrating steps for the multi-part absorbing grating as in Figs. 7 and 8.

### DETAILED DESCRIPTION OF EMBODIMENTS

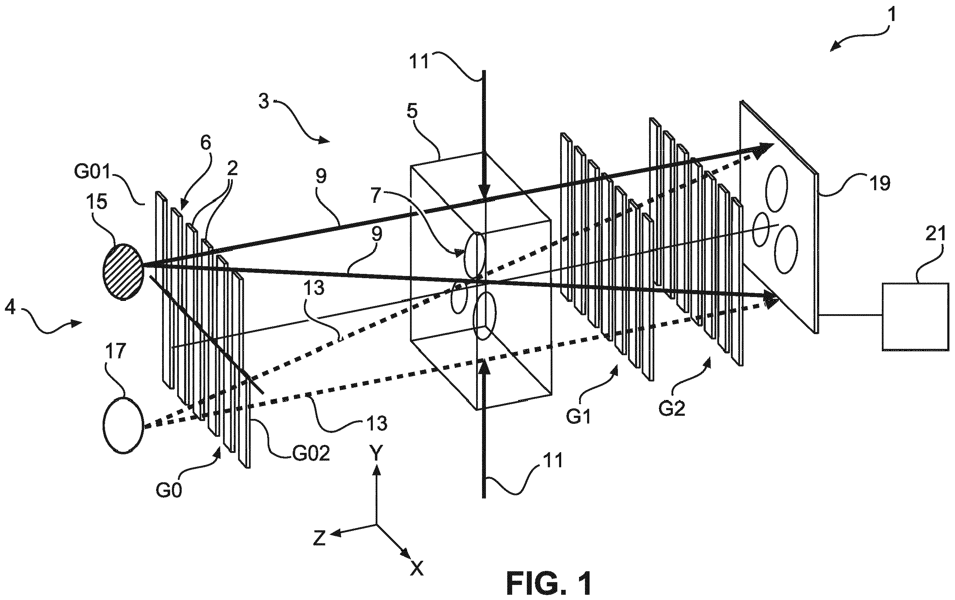

Fig. 1 shows a multi-spot X-ray imaging system 1 comprising a multi-spot X-ray device 3. The multi-spot X-ray device 3, comprises a multi-spot array 4 providing at least two focal spots 15, 17 configured to emit X-rays 9, 13. The multiple focal spots 15, 17 are arranged in a linear arrangement, essentially on top of each other, seen in y-direction (cf. depicted coordinate system). The field of view 11, shown by two arrows 11, is the dimension parallel to a line between the focal spots 15, 17. In this embodiment the focal spots 15, 17 have the same distance (in z-direction) from the multi-part absorbing grating G0. The focal spots 15, 17 emit X-rays 9, 13 in a subsequent manner. One of the focal spot 15, 17 emits X-rays 9, 13 at a time. The emitted X-rays 9 or 13 are transmitted by an object 7 and gratings G0, G1, G2. The focal spots 15, 17 can be configured to emit coherent X-rays 9, 13.

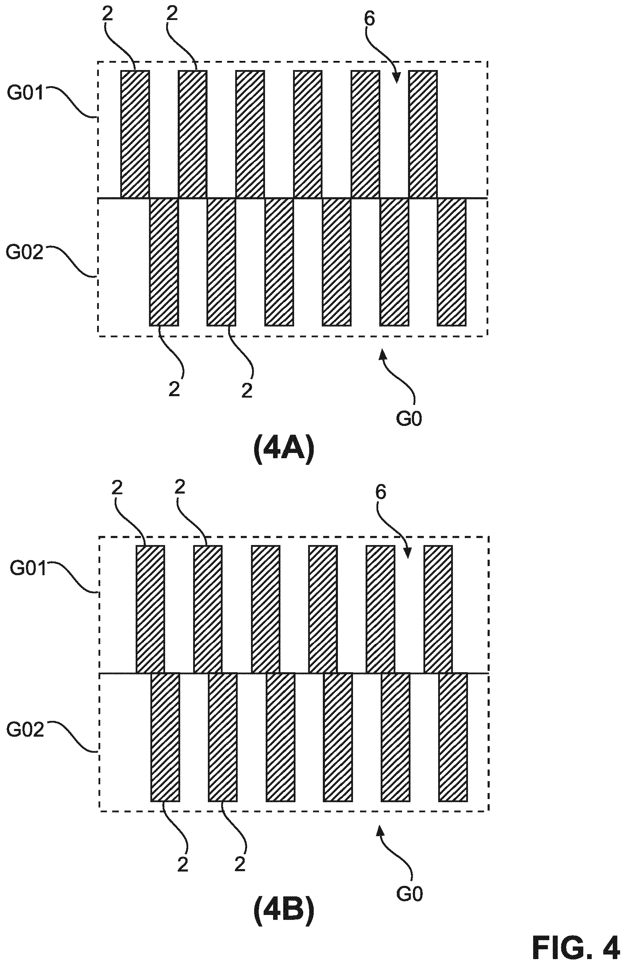

A multi-part absorbing grating G0 is configured to transmit the X-rays through at least one of its two parts G01, G02, in this embodiment. In other embodiments, the multi-part absorbing grating can provide more than two parts G01, G02. The two parts G01, G02 of the multi-part absorbing grating G0 are provided with a phase offset 4 relative to each other. This means that the grating bars 2 of the upper part G01 are provided with a phase offset 6 relative to the lower part G02 (which is more apparent in the view of Fig. 4A, 4B for example). Thus, the stepping speed of the grating G0 can be reduced by have compared to conventional stepping rates.

A non-absorbing first grating G1 and an absorbing a second grating G2 are located behind an object-receiving region 5 arranged between the multi-part absorbing grating G0 and the first grating G1. In this embodiment, the multi-part absorbing grating G0 provides a phase offset relative to the first grating G1 and/or second grating G2. Such arrangement provides differential phase contrast imaging (DPCI) and dark-field imaging (DFI).

The system 1 comprises an X-ray source configured to provide at least one X-ray beam. The X-rays 9, 13 are emitted by the focal spots 15, 17. The system 1 further comprises a processing unit 21 configured to integrate data signals provided by the detector 19. The detector 19 receives the X-rays 9, 13 transmitted by the object 7 and the gratings G0, G1, G2. The received X-rays 9, 13 correspond to those emitted by the focal spots 15, 17. The data signals are integrated by the processing unit 21 which produces an image of the object 7 by overlaying the integrated data signals. In this embodiment, the detector 19 is a digital detector.

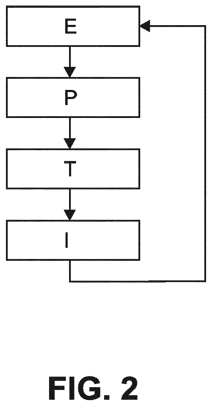

Fig. 2 shows a flow chart of a method for imaging an object by use of a multi-spot X-ray device 3. The method comprises several steps. Emitting X-rays, step E, subsequently from at least two focal spots 15, 17 of a multi-spot array 4. Passing the X-rays, step P, through at least one of the parts G01, G02 of a multi-part absorbing grating G0. Transmitting the X-rays, step T, through the object 7, a first grating G1 and a second grating G2 and integrating, step I, data signals provided by a detector 19 corresponding to X-rays 9, 13 subsequently emitted by the at least two focal spots 15, 17.

As described for Fig. 1, at least two of the parts G01, G02 of the multi-part absorbing grating G0, are provided with a phase offset 6 relative to each other. Only one focal spot 15, 17 emits X-rays 9, 13 at a time. The other focal spots 15, 17 of the multi-spot array 4 is off. The emitted X-rays 9, 13 pass through one of the grating parts G01, G02 provided by the multi-part absorbing grating G0. After the data signals provided by the detector are integrated for the X-rays received by the detector 19 from one focal spot 15, 17, the remaining focal spot 15, 17 emits X-rays which pass through one of the remaining parts G01, G02 of the multi-part absorbing grating G0. The illuminated part G01, G02 of the multi-part absorbing grating G0 provides a phase offset 6 compared to the before illuminated part G01, G02 of the multi-part absorbing grating G0.

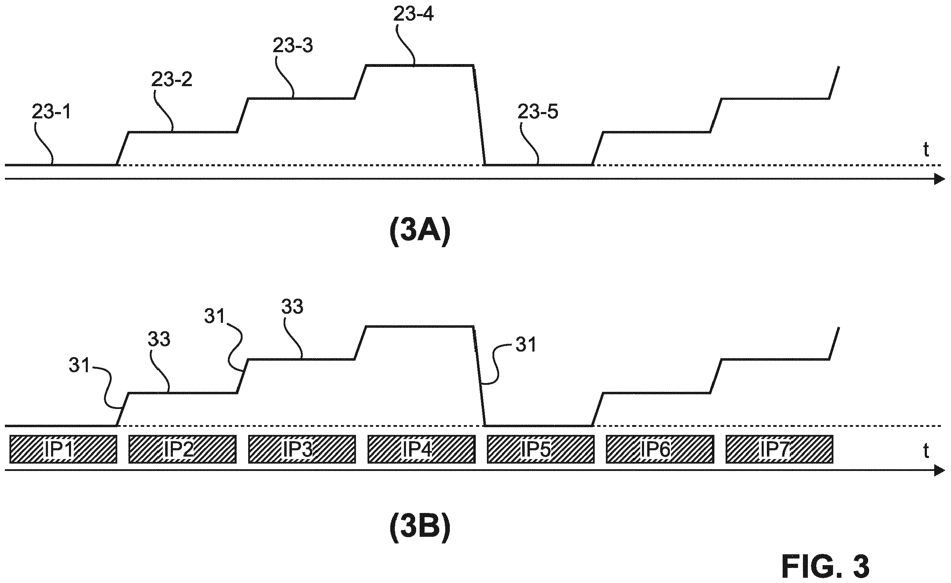

Fig. 3 is an example of stepping a multi-part absorbing grating G0 providing a single part. Thus, there is no phase offset.

The upper part 3A of Fig. 3 shows the phase of the multi-part absorbing grating G0 relative to the gratings G1 and G2. The phase is shown as a function of time t.

The lower part 3B of Fig. 3 shows the physical position of the multi-part absorbing grating G0 over time t including integration periods IP1 ... IP7.

As the multi-part absorbing grating G0 provides only a single part, phase stepping is required on every reading of the X-rays passing the object 7 and the gratings G1, G2. IP1 ... IP7 refers to integration period, i.e. the time that the data signal received by the detector is integrated.

In this embodiment, an example for phase stepping with four grating positions is shown. During integration period IP1, phase 23-1 is provided by the multi-part absorbing grating G0 relative to the gratings G1 and G2. After integration completion, the multi-part absorbing grating G0 is physically moved 31 to the next position 33, in order to provide phase 23-2. Integration is performed during integration period IP2. This process is repeated for integration periods IP3 and IP4 for offset 23-3 and 23-4. After each integration period, the multi-part absorbing grating G0 is physically moved 31 to the next position 33. The multi-part absorbing grating G0 motion must be strictly synchronized with IPs. This means that the grid motion must be fast as, otherwise, the fringe pattern is smeared out.

Before integration period IP5, after stepping four gratings positions, the phase 23-5 is equal to the starting phase 23-1 and the physical position of the multi-part absorbing grating G0 is as in the beginning of the process. However, this time the motion 31 of the grating G0 is larger as for the previous motions of the grating G0, as can be seen in the lower part 3B of Fig. 3. After integration period IP5 the phase stepping with four grating positions starts all over.

Fig. 4 shows an embodiment of a multi-part absorbing grating G0 providing a phase offset of 180 degrees (left part 4A) and a phase offset of 120 degrees (right part 4B). The multi-part absorbing grating G0, also called source grating, has two parts or areas G01, G02. One part G01 is illuminated by focal spot 15 (Fig. 1), the other part G02 by focal spot 17 (Fig. 1). The grating bars 2 of the two parts G01, G02 have a phase offset 6 with respect to each other.

In the left part 4A of Fig. 4 the phase offset 6 is 180 degrees. In the right part 4B of Fig. 4 the phase offset 6 is 120 degrees.

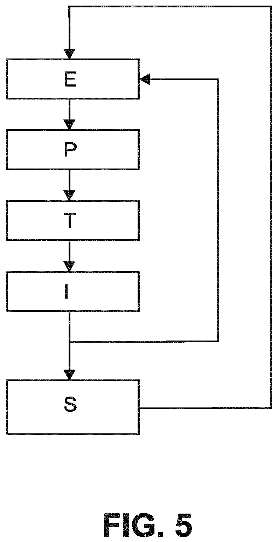

Fig. 5 shows a flow chart of the method for imaging an object including stepping of the multi-part absorbing grating G0 of Fig 4, for example.

X-rays are subsequently emitted, step E, from at least two focal spots 15, 17 of a multi-spot array 4 (cf. Fig. 1 for the multi-spot X-ray device). The X-rays are passed, step P, through at least one of the parts of the multi-part absorbing grating of Fig. 4, for example, and transmitted, step T, through the object 7, a first grating G1, and a second grating G2. The data signals provided by a detector 19 are integrated, step I.

Only one focal spot 15, 17 emits X-rays 9, 13 at a time. The other focal spots of the multi-spot array is(are) off. After the data signals provided by the detector 19 are integrated for the X-rays received from one of the focal spots 15, 17, the remaining focal spot 15, 17 emits X-rays 9, 13 which pass, step P, through the remaining part G01, G02 of the multi-part absorbing grating G0, the object 7 and the gratings G1, G2. The corresponding signals are integrated, step I.

After integration of the data signals corresponding to the X-rays 9, 13 emitted by all focal spots 15, 17, the multi-part absorbing grating G0 is stepped, step S. In other words, there is no need for stepping the multi-part absorbing grating G0 after each integration period. The multi-part absorbing grating G0 remains in position as long as the focal spots 15, 17 are emitting X-rays 9, 13 in an alternating manner.

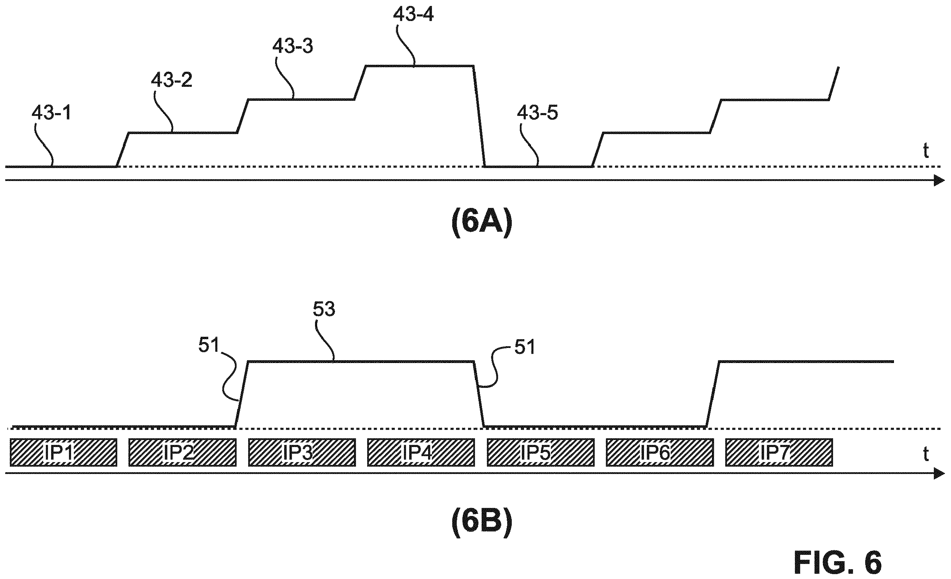

Such method reduces the stepping speed as well as the stepping steps. Each integration of the data signals is performed during a corresponding integration period, cf. Fig. 6. In embodiments with more than two focal spots, e.g. three focal spots, all three or more focal spots emit X-rays which pass through the corresponding part of the multi-part absorbing grating, are transmitted through the object and the first and second grating.

Fig. 6 shows stepping and integrating steps for a multi-part absorbing grating providing a phase offset of 90 degrees between its two parts G01, G02. Similar stepping and integrating steps apply to the multi-part absorbing gratings G0 shown in Fig. 4.

The upper part 6A of Fig. 6 shows the phase of the multi-part absorbing grating G0 relative to the gratings G1 and G2 (cf. Fig.1). The phase is shown as a function of time t. The lower part 6B of Fig. 6 shows the physical position of the multi-part absorbing grating G0 over time t including integration periods IP1 ... IP7. IP1 ... IP7 refers to integration period, i.e. the time that the data signal received by the detector is integrated.

The multi-part absorbing grating G0 provides two parts G01, G02 which are alternatingly illuminated by X-rays 9, 13 emitted by the focal spots 15, 17. As can easily be seen from the lower part 6B, only half the number of steps are needed for the physical motion of the multi-part absorbing grating G0. The phase offset between the multi-part absorbing grating G0 and the gratings G1, G2 changes while the position of the multi-part absorbing grating G0 remains unchanged.

In this embodiment, a phase stepping with two grating positions is shown. During integration period IP1 and IP2, the multi-part absorbing grating G0 is not moved, while two different phases 43-1 and 43-2 are provided, respectively, by the multi-part absorbing grating G0 relative to the gratings G1 and G2.

After integration completion of the two integration periods IP1, IP2, the multi-part absorbing grating G0 is physically moved 51 to the next position 53, in order to provide phases 43-3 and 43-4, respectively. In this embodiment, the multi-part absorbing grating G0 is physically moved 51 to the next position 53 after each second integration period only. Thus, the stepping steps of the multi-part absorbing grating G0 are reduced by half.

Before integration period IP5, after stepping two gratings positions, the phase 43-5 is equal to the starting phase 43-1 and the physical position of the multi-part absorbing grating G0 is as in the beginning of the process. After integration period IP5 the phase stepping with two grating positions starts all over.

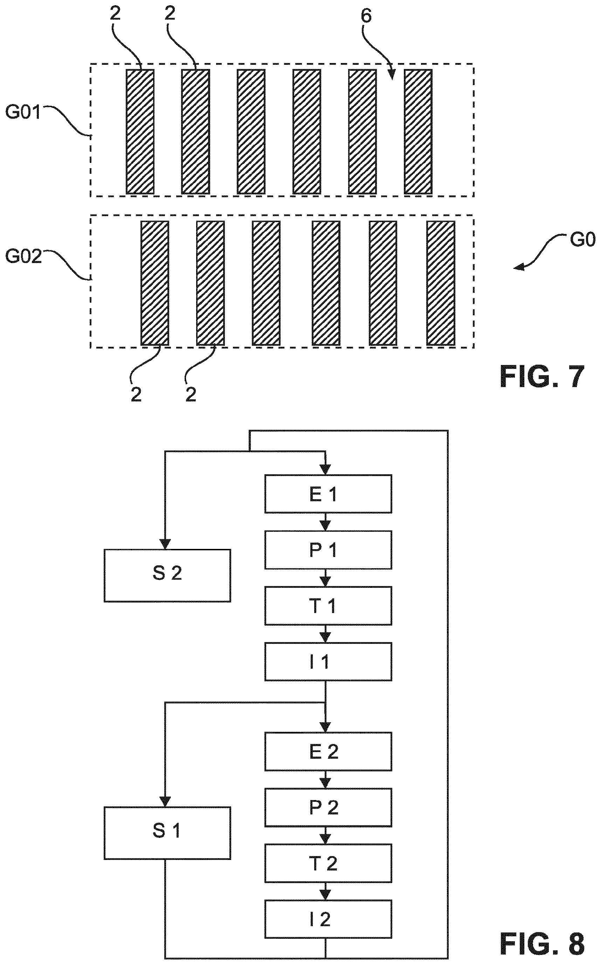

Fig. 7 shows a multi-part absorbing grating G0 provided as two separate gratings G01, G02. The multi-part absorbing grating G0, also called source grating, physically split in two separate parts or areas G01, G02. One part G01 is illuminated by focal spot 15 (Fig. 1), the other part G02 by focal spot 17 (Fig. 1). The grating bars 2 of the two parts G01, G02 have a phase offset 6 of 120 degrees with respect to each other. The two separate parts G01, G02 can be moved independently from each other.

Fig. 8 shows a flow chart of a method for imaging an object by using the multi-part absorbing grating of Fig. 7. The method is described for two focal spots for the sake of understanding. However, the same applies for embodiments with more than two focal spots.

X-rays are emitted, step E1, from focal spot 15 of a multi-spot array 4 (cf. Fig. 1 for the multi-spot X-ray device). The X-rays are passed, step P1, through the part G01 of the multi-part absorbing grating G0 of Fig. 7, and transmitted, step T1, through the object 7, a first grating G1, and a second grating G2. The data signals provided by a detector 19 are integrated, step I1.

The following steps are performed simultaneously. Focal spot 17 emits X-rays, step E2, which pass, step P2, through the remaining part G02 of the multi-part absorbing grating G0, and are transmitted, step T2, by the object 7 and the gratings G1, G2. The corresponding signals are integrated, step I2. Simultaneously, part G01 of the multi-part absorbing grating G0 is physically moved, step S1, to provide a different phase for the next integration period, during which focal spot 15 emits X-rays, etc., steps E1, P1, T1, I1.

After integration of the data signals corresponding to the X-rays emitted by focal spots 17 and simultaneously stepping part G01 of the multi-part absorbing grating G0, focal spot 15 emits X-rays, etc. and simultaneously, part G02 is physically moved, step S2, to provide a different phase for the next integration period, during which focal spot 17 emits X-rays, etc., steps E2, P2, T2, I2.

In other words, during the integration period for one focal spot and the corresponding part of the multi-part absorbing grating G0, the other part of the grating is moved in position as the two parts are separated.

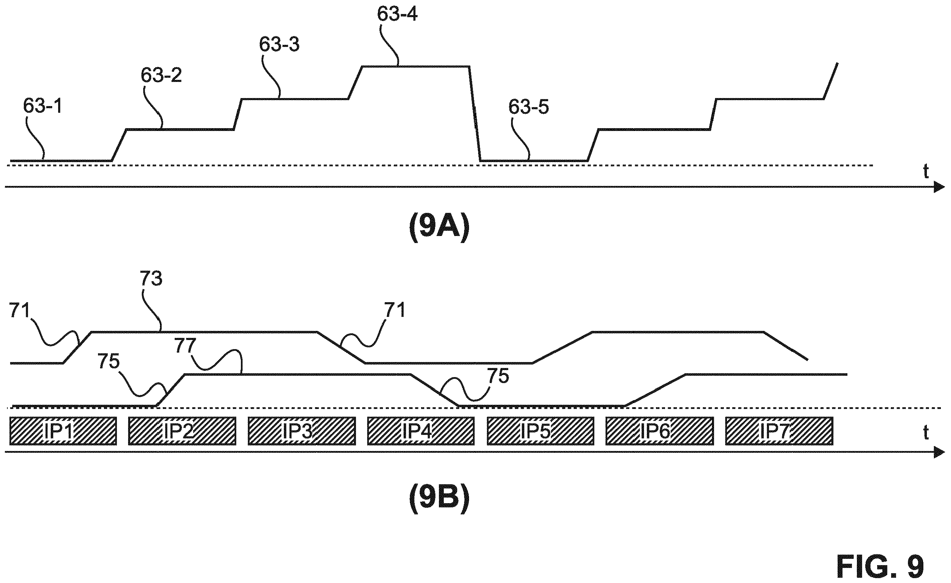

Such method reduces the stepping speed as well as the stepping steps and provides independent motion for each part of the grating G0 during each integration of the data signals which is performed during a corresponding integration period, cf. Fig. 9. In embodiments with more than two focal spots, e.g. three focal spots, all three or more focal spots emit X-rays which pass through the corresponding (separate) part of the multi-part absorbing grating, are transmitted through the object and the first and second grating.

Fig. 9 shows stepping and integrating steps for the multi-part absorbing grating as in Figs. 7 and 8. The upper part 9A of Fig. 9 shows the phase of the multi-part absorbing grating G0 relative to the gratings G1 and G2 (cf. Fig.1). The phase is shown as a function of time t. The lower part 9B of Fig. 9 shows the physical position of the multi-part absorbing grating G0 over time t including integration periods IP1 ... IP7. IP1 ... IP7 refers to integration period, i.e. the time that the data signal received by the detector is integrated.

The multi-part absorbing grating G0 provides two separate parts G01, G02 which are alternatingly illuminated by X-rays 9, 13 emitted by the focal spots 15, 17. Each part G01, which in this embodiment are separate gratings G01, G02, is moved during the integration time of the other part G02.

As can easily be seen from the lower part 9B, the non-illuminated grating G01, G02 is moved freely during the integration period of the illuminated grating G01. Different phases 63-1 ... 63-4 between the multi-part absorbing grating G0, in this embodiment comprising the two separate gratings G01, G02, and the gratings G1, G2 are provided (upper part 9A) for corresponding integration times over time (lower part 9B).

During integration period IP1, where part G01 is illuminated and the X-rays of focal spot 15 are transmitted, detected and the data signal is integrated, the other part G02 is moved 71 into position 73. The phase 63-1 relative to the gratings G1 and G2 is provided.

After integration completion for integration period IP1, because part G02 is already in position 73, it can be immediately illuminated by the X-rays emitted by focal spot 17 during integration period IP2, where a phase 63-2 is provided. The X-rays of focal spot 17 are transmitted, detected and the data signal is integrated. At the same time part G01 is moved 75 in position 77.

The alternating emission E1, E2 of the X-rays by the focal spots 15, 17 followed by passing P1, P2 transmission T1, T2 and detection of the X-rays and the integration I1, I2 of the corresponding data signals is performed as long as required.

Such method is far less demanding with respect to synchronization. Also, it is far less demanding with respect to stepping speed, as the movement of the non-illuminated separate grating G01, G02 can by anywhere in the integration period of the other separate grating G01, G02.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A multi-spot X-ray device, comprising:
- a multi-spot array providing at least two focal spots configured to emit X-rays,
- a multi-part absorbing grating configured to transmit the X-rays through at least one of its parts,
- a first grating and a second grating; and
- an object-receiving region arranged between the multi-part absorbing grating and the first grating or the first grating and the second grating,
wherein at least two parts of the parts of the multi-part absorbing grating are provided with a phase offset relative to each other.

2. Multi-spot X-ray device according to claim 1, wherein the at least two focal spots of the multi-spot array are arranged facing at least a first and a second part, respectively, of the multi-part absorbing grating.

3. Multi-spot X-ray device according to one of the claims 1-2, wherein the focal spots are configured to alternatingly or sequentially emit X-rays.

4. Multi-spot X-ray device according to one of the claims 1-3, wherein the phase offset is between 0 and 180 degrees.

5. Multi-spot X-ray device according to one of the claims 1-4, wherein the multi-part absorbing grating is configured to be rotated.

6. Multi-spot X-ray device according to one of the claims 1-5, wherein at least one of the parts of the multi-part absorbing grating is provided as separate grating.

7. Multi-spot X-ray device according to one of the claims 1-6, wherein the multi-spot array or the at least two focal spots are configured to be arranged, so that the X-rays emitted by one of the focal spots illuminates one of the parts of the multi-part absorbing grating.

8. A multi-spot X-ray imaging system, comprising:
- a source configured to provide at least one X-ray beam;
- a multi-spot X-ray device comprising multiple focal spots and a multi-part absorbing grating, wherein the focal spots are configured to emit X-rays;
- a detector configured to receive X-rays emitted from at least a part of the multiple focal spots and transmitted at least through one of parts of the multi-part absorbing grating; and
- a processing unit configured:
- to integrate data signals provided by the detector corresponding to X-rays emitted by the multiple focal spots; and
- to produce an image of an object by overlaying the integrated data signals; wherein at least two of the parts of the multi-part absorbing grating are provided with a phase offset relative to each other.

9. A method for imaging an object by use of a multi-spot X-ray device, the method comprising the following steps:
- emitting X-rays subsequently from at least two focal spots of a multi-spot array;
- passing the X-rays through at least one of the parts of a multi-part absorbing grating;
- transmitting the X-rays through the object, a first grating and a second grating; and
- integrating data signals provided by a detector corresponding to X-rays subsequently emitted by the at least two focal spots;
wherein at least two of the parts of the multi-part grating are provided with a phase offset relative to each other.

10. Method according to claim 9, comprising stepping the multi-part grating after integration of the data signals provided by the detector corresponding to the X-rays emitted by the at least two focal spots.

11. Method according to claim 10, comprising stepping the multi-part absorbing grating by rotation of the multi-part absorbing grating.

12. Method according to one of the claims 9 - 11, wherein at least one of the parts of the multi-part absorbing grating is provided as separate absorbing grating and wherein the at least one part is being stepped during integration of the data signals provided by the X-rays passing another part of the multi-part absorbing grating.

13. Method according to one of the claims 9 - 12, wherein during transmission of X-rays from one of the focal spots only one of the parts of the multi-part absorbing grating is illuminated.

14. A computer program element for controlling an apparatus according to one of the claims 1 to 8, which, when being executed by a processing unit, is adapted to perform the method steps of one of the claims 9 to 13.

15. A computer readable medium having stored the program element of claim 14.
